# EUROPÄISCHE PATENTSCHRIFT

(11) **EP 0 697 860 B1**
(45) Veröffentlichungstag und Bekanntmachung des Hinweises auf die Patenterteilung: **11.10.2000**
(21) Anmeldenummer: 94915533.7
(22) Anmeldetag: 25.04.1994
(51) Int. Cl.: A61K 9/70, A61K 31/565

(54) **ESTRADIOLHALTIGES TRANSDERMALES THERAPEUTISCHES SYSTEM**
TRANSDERMAL THERAPEUTIC SYSTEM CONTAINING ESTRADIOL
SYSTEME THERAPEUTIQUE TRANSDERMIQUE CONTENANT DE L'OESTRADIOL

(30) Priorität: 06.05.1993 DE 4314970; 27.10.1993 DE 4336557
(43) Veröffentlichungstag der Anmeldung: 28.02.1996
(73) Patentinhaber: LTS Lohmann Therapie-Systeme AG, 56626 Andernach (DE); MERCK PATENT GmbH, 64271 Darmstadt (DE)
(72) Erfinder: MECONI, Reinhold, D-56567 Neuwied (DE); SEIBERTZ, Frank, D-53557 Bad Hönningen/Ariendorf (DE); HORSTMANN, Michael, D-56564 Neuwied (DE); LICHTENBERGER, Rainer, D-64289 Darmstadt (DE)
(74) Vertreter: Flaccus, Rolf-Dieter, Dr.
(86) Internationale Anmeldenummer: EP9401279
(87) Internationale Veröffentlichungsnummer: WO9426257

(56) Entgegenhaltungen:
- EP-A- 0 285 563
- EP-A- 0 607 434
- DE-C- 4 223 360

## Beschreibung

Die Erfindung betrifft ein transdermales therapeutisches System zur kontrollierten Abgabe von Estradiol oder seinen pharmazeutisch unbedenklichen Derivaten alleine oder in Kombination mit Gestagenen wie Levonorgestrel an die menschliche oder tierische Haut, seine Verwendung und ein Verfahren zu seiner Herstellung.

Transdermale therapeutische Systeme (TTS) sind in der Therapie einer Reihe von Erkrankungen bereits im Markt eingeführt. Auch transdermale therapeutische Systeme mit dem estrogenen Wirkstoff 17-β-Estradiol sind als Therapeutikum für Wechseljahrsbeschwerden und neuerdings auch gegen Osteoporose im Handel und erfolgreich in der Therapie.

Levonorgestrel ist ein synthetisches Gestagenderivat, das in Kombination mit oral wirksamen Estrogenen bisher vor allem in Kontrazeptiva eingesetzt wurde. Gestagene, also auch Levonorgestrel, haben in solchen Präparaten die Funktion, durch entsprechende trophische Vorbereitung des Uterus eine möglichst kurze, schnelle und "physiologische" Entzugsblutung folgen zu lassen. Es gibt auch Hinweise auf eine Schutzwirkung der Gestagenzugabe vor dem Risiko endometrialer Tumoren.

Es ist daher sinnvoll, auch für die Indikation postmenopausaler Beschwerden zu einer zyklischen Behandlungsweise zu kommen d.h. zu einer zeitweisen festen Arzneimittelkombination aus Estrogenen (z.B. Estradiol) und Gestagenen (z.B. Levonorgestrel). Besonders attraktiv ist eine solche Kombination beider Wirkstoffe in einem gemeinsamen, monolithischen transdermalen therapeutischen System, das nur einmal am Tag oder sogar nur ein- bis zweimal pro Woche zu applizieren wäre. Aufgrund seiner hohen Wirksamkeit und Permeationsfähigkeit durch die Haut ist Levonorgestrel für ein solches System hervorragend geeignet.

In der Literatur sind experimentelle Systeme zur transdermalen Zufuhr von Levonorgestrel beschrieben (Friend et. al., J. Controlled Release 7, 243-250 (1988)). Allerdings sind nach dieser Einschätzung Permeationsverbesserer (Enhancer), z.B. Alkylester kurzfettiger Fettsäuren, zur erfolgreichen transdermalen Therapie mit ausreichend kleiner Systemfläche erforderlich (Friend et. al., J. Controlled Release 9, p33-40 (1989)).

Für die transdermale Anwendung von Estrogenen und Gestagenen sind bereits eine Reihe von Vorrichtungen bekannt geworden. Unter Verwendung von Styrol-Isopren-Blockcopolymer, feuchtigkeitsabsorbierenden Polymerdomänen und dem Enhancer (und Juckreizstiller) Crotamiton gelangten Nakagawa et al. (EP-A 0 483 370) zu einem Matrix-Transdermalen therapeutischen System für Estradiol allein. Eine andere Konzeption liegt in der gleichzeitigen Applikation von Estradiol und einem Enhancer (Ethanol) in einem membrangesteuerten Reservoirsystem (Campbell et al. US PS 4 379 454) vor, das auch in einer Kombinationsarzneiform mit dem Gestagen Norethisteronacetat verwendbar (Fankhauser und Schenkel, DE 3 810 896) ist.

Transdermale therapeutische Systeme zur Abgabe von Estradiol und/oder Gestagenen weisen jedoch die Nachteile auf, daß sie entweder Ethanol enthalten oder die potentielle Gefahr besitzen, daß der Wirkstoff im Laufe der Zeit rekristallisiert.

Aus der DE-OS 32 05 258 und der EP 0 285 563 ist bekannt, Estradiol und Ethanol gleichzeitig in einer Pflasterformulierung zu verabreichen. Die Herstellung dieses Pflasters ist jedoch sehr aufwendig und der Tragekomfort nach Applikation aufgrund der fehlenden Flexibilität gering.

Die EP 0 285 563 beschreibt ein transdermales therapeutisches System für die kombinierte Applikation von Oestrogenen und Gestagenen. Das Reservoir erhält die Wirkstoffformulierung, gegebenenfalls eine Membran sowie Ethanol als perkutanes absorbtionsverbesserndes Mittel. Da die Freisetzung des Wirkstoffes hauptsächlich von der Membran gesteuert wird, unterscheidet sich dieses transdermale therapeutische System grundsätzlich von dem Wirkstoff-pflaster gemäß der vorliegenden Erfindung. Der Kleber hat bei dem dort beschriebenen Pflaster lediglich die Funktion, das Pflaster auf der Haut zu befestigen. Daß er einen Beitrag zur Steuerung der Wirkstofffreisetzung zu leisten vermag, ist nicht seine Hauptaufgabe, sondern lediglich ein - möglicherweise sogar unerwünschter - Nebeneffekt. Es handelt sich hierbei um ein sogenanntes "Beutelpflaster", da sich die Wirkstoffzubereitung in einem Beutel, bestehend aus undurchlässiger Rückschicht und Membran mit Kleberschicht befindet. In Folge seines komplizierten Aufbaues ist die Herstellung des Pflasters sehr aufwendig, da die Einzelkomponenten separat hergestellt und dann in einem weiteren Arbeitsgang zu einem Pflaster zusammengefügt werden müssen.

Die EP 0 275 716 beschreibt ein - im Gegensatz zu dem erfindungsgemäßen einschichtigen System - zweischichtiges transdermales System zur simultanen Verabreichung von einem oder mehreren Oestrogenen, die in der Polymerschicht gelöst oder microdispergiert sind. Die Haftschicht enthält dabei außer den Wirkstoffen Substanzen, die die transdermale Absorption verbessern. Polymer- und Haftschicht können aus Polyacrylaten, Silikonen oder Poliisobutylenen bestehen.

In der EP 0 072 251 ist eine flexible, flüssigkeitsabsorbierende, medikamentöse Bandage beschrieben. Das an der flexiblen Rückschicht befestigte Substrat besteht aus einer hydrophilen Matrix auf der Basis von hydrophilen hochmolekularen Polysacchariden und/oder Polyacrylsäure, Polyacrylamid, Ethylen-Vinylacetat-Copolymeren und anderen Polymeren sowie einer flüssigen Phase auf der Basis einer Lösung oder Emulsion aus Kohlehydrat, Proteinen und mehrwertigen Alkoholen und verschiedenen Wirkstoffen, u.a. auch Hormonen. Wesentliches Merkmal dieser Erfindung ist der feuchttigkeitsabsorbierende Kleber.

Die EP 0 328 806 beschreibt ein membranfreies, transdermales therapeutisches System, dessen Matrix aus einem Polyacrylatkleber, einem Lösemittel, einem Penetrationsverbesserer und Oestrogenen, dessen Derivaten und Kombinationen davon besteht.

In der WO 87/07 138 ist ein Estradiol-Pflaster auf der Basis einer Rückschicht, einer den Wirkstoff enthaltenden Matrix und einem Haftkleber, der mit einer wiederablösbaren Schutzschicht abgedeckt ist, beschrieben. Die Herstellung von Matrix und Haftkleber erfolgen in technologisch sehr aufwendigen Arbeitsvorgängen durch Homogenisieren, Entgasen, Beschichten, Trocknen und Vereinzeln. In einer Ausführungsform muß die Rückschicht sogar mit einem Haftkleber beschichtet werden, was einen weiteren Arbeitsgang bedingt. Das Zusammenfügen der einzelnen Teile erfolgt in einem separaten Arbeitsgang. Die Herstellung des Pflasters ist also insgesamt sehr aufwendig und kompliziert.

Aus der US-PS 4 624 665 sind Systeme bekannt, die im Reservoir den Wirkstoff in mikroverkapselter Form enthalten. Das Reservoir ist eingebettet zwischen Rückschicht und einer Membran. Der äußere Rand des Systems ist mit einem Haftkleber ausgerüstet. Der Aufbau und die Herstellung dieses Systems ist sehr kompliziert, da der Wirkstoff mikroverkapselt und in einer flüssigen Phase homogen verteilt werden muß, die dann in weiteren Arbeitsgängen zwischen Rückschicht und Membran eingebettet wird. Zusätzlich muß das System dann mit einem klebenden Rand versehen und mit einer Schutzschicht abgedeckt werden.

Es sind weiterhin aus der EP 0 186 019 Wirkstoffpflaster bekannt, bei denen einer Kautschuk-/Klebharzmasse in Wasser quellbare Polymere zugesetzt sind und aus denen Estradiol freigesetzt werden kann. Es hat sich jedoch gezeigt, daß die Freisetzung von Estradiol aus diesen Wirkstoffpflastern viel zu gering ist und nicht den therapeutischen Erfordernissen entspricht.

In der DE-OS 20 06 969 ist ein Pflaster oder ein Haftverband mit Systemwirkung beschrieben, bei dem empfängnisverhütende Substanzen in die Klebstoffkomponente oder den Klebstoffilm eingearbeitet werden. Aus dieser Schrift ist zu entnehmen, daß der Klebstoff ein Acrylat sein kann.

Die DE-OS 39 33 460 beschreibt ein oestrogenhaltiges Wirkstoffpflaster auf der Basis von Homo- und/oder Copoly-meren mit mindestens einem Derivat der Acryl- oder mit Methacrylsäure in Kombination mit in Wasser quellbaren Substanzen.

Es hat sich jedoch gezeigt, daß haftklebende transdermale therapeutische Matrix-Systeme, die den Wirkstoff teilweise oder vollständig gelöst enthalten, die potentielle Gefahr beinhalten, daß der Wirkstoff im Laufe der Zeit rekristallisiert. Dadurch sinkt die Wirkstofffreisetzung und das oestrogenhaltige Pflaster entspricht nicht mehr den therapeutischen Erfordernissen.

Ein weiterer Nachteil dem Stand der Technik entsprechender Systeme ist der Einsatz von Enhancern, welcher eine grundsätzlich unerwünschte zusätzliche Beeinflussung der Haut mit dem Risiko der Irritation mit sich bringt. Weitere Nachteile liegen in dem aufwendigen Aufbau solcher Systeme (Einsatz mehrerer wirkstoffhaltiger Schichten, Verwendung von Steuermembranen), die das fertige Produkt beim Verwender in der Regel unakzeptabel machen.

Es ist somit Aufgabe der Erfindung, die oben angeführten Nachteile zu vermeiden und ein stabiles, d.h. rekristallisationsfreies, oestrogenhaltiges Pflaster bzw. transdermales therapeutisches System zur Verfügung zu stellen, dessen Freisetzung sich durch Lagerung nicht verändert, wobei dessen Aufbau möglichst dünn zu gestalten ist und bei dessen therapeutischem Einsatz die Haut über die Wirkstoffe Estradiol und Gestagen hinaus nicht mit hautbeeinflussenden Stoffen (Enhancern) behandelt wird.

Überraschenderweise hat sich gezeigt, daß die Aufgabe dadurch gelöst wird, daß der oestrogenhaltige Haftkleber überwiegend aus Estern des Kolophoniums aufgebaut ist.

Von Vorteil ist hierbei die zusätzliche Verwendung eines Styrol-Isopren-Blockcopolymers und hydrierter Harzsäuren oder deren Derivate in der aktiven Schicht, welche beispielsweise eine therapeutisch erforderliche Menge der Wirkstoffe Estradiol und Levonorgestrel enthält.

Eine Kombination der beiden Hilfsstoffe, des Styrol-Isopren-Block-Oopolymers, das als kohäsive Komponente dient, und der hydrierten Harzsäuren oder deren Derivate, die als klebrigmachende Stoffe fungieren, ergibt nicht nur einen gut klebrigen und kohäsiven Kautschukkleber, sie stellt darüberhinaus herausragende biopharmazeutische Eigenschaften bei, insbesondere gute Hautverträglichkeit und Permeationsfähigkeit, sowie eine Vermeidung der Rekristallisation der Wirkstoffe.

Die Erfindung betrifft somit ein transdermales therapeutisches System zur kontrollierten Abgabe von Estradiol oder seinen pharmazeutisch unbedenklichen Derivaten allein oder in Kombination mit Gestagenen, bestehend aus einer Rückschicht, einem damit verbundenen wirkstoffhaltigen Reservoir, das unter Verwendung von Haftklebern hergestellt ist, und einer wiederablösbaren Schutzschicht, wobei der Haftkleber Ester des Kolophoniums und Hilfsstoffe enthält.

Zu den Estern des Kolophoniums gehören z.B. Methylester, der Glycerinester, der Pentaerythritester, der maleinsäuremodifizierte Pentaerythritester, der maleinsäuremodifizierte Glycerinester und der Triethylenglycolester. Der Anteil an Estern des Kolophoniums in dem estradiolhaltigen Haftkleber beträgt 55-92 Gew.-% , vorzugsweise 60-90 Gew.-% und ganz besonders bevorzugt 70-88 Gew.-%. Desweiteren kann der Haftkleber Ester des hydrierten Kolophoniums enthalten. Besonders bevorzugte Ester des Kolophoniums sind der Triethylenglycolester, der Glycerinester und der Pentaerythritester des hydrierten Kolophoniums.

In einer weiteren Ausführungsform können weitere Bestandteile des estradiolhaltigen Haftklebers Polymere sein, die ausgewählt sind aus der Gruppe der Styrol-Butadien-Styrol-Blockeopolymere, Styrol-Isopren-Styrol-Blockcopolymere, Styrol-Ethylen-Butylen-Styrol-Blockcopolymere, Ethylen-Vinylacetat-Copolymere, Polyvinylpyrrolidon, Cellulosederivate und Polymere auf der Basis von Acrylsäure- und Methacrylsäurederivaten. Diese Polymere sind in einer Konzentration von 6-25 Gew.-% in der estradiolhaltigen Klebmasse enthalten.

Das rekristallistaionsfreie estradiolhaltige Pflaster enthält im Reservoir Estradiol und seine pharmazeutisch unbedenklichen Derivate allein oder in Kombination mit Gestagenen in einer Konzentration von insgesamt 2-15 Gew.-%, und zwar in einem molaren Verhältnis von 1 : 1 bis 1 : 10.

Das estradiolhaltige Reservoir kann mindestens einen Bestandteil der Gruppe enthalten, welche Alterungsschutzmittel, Weichmacher, Antioxidantien und Absorbtionsverbesserer umfaßt. Geeignete Weichmacher sind dem Fachmann bekannt und beispielsweise in der DE 37 43 949 beschrieben. Das estradiolhaltige Reservoir enthält üblicherweise Weichmacher in einem Anteil von 0-5 Gew.-%.

Weiterhin sind im wirkstoffhaltigen Reservoir auch Alterungsschutzmittel in einer Konzentration von 0-1 Gew.-% enthalten. Diese sind dem Fachmann bekannt und Z.B. in der DE 37 43 946 beschrieben.

Das estradiolhaltige Reservoir kann sowohl aus Lösung als auch aus der Schmelze erzeugt werden.

Für den Fall, daß das Reservoir keine ausreichende Eigenklebrigkeit zur Haut aufweist, kann dieses mit einer Haftkleberschicht oder mit einem haftklebenden Rand versehen werden. Auf diese Weise ist es gewährleistet, daß das transdermale Pflaster über den gesamten Applikationszeitraum auf der Haut haftet.

Ein besonders bevorzugter Aufbau des transdermalen estradiolhaltigen Pflasters ist das Matrix-System, bei dem bekanntlich die Matrix die Steuerung für die Wirkstofffreisetzung übernimmt und diese dem Vt-Gesetz nach Higuchi gehorcht. Das bedeutet jedoch nicht, daß nicht in besonderen Fällen auch das Membran-System angezeigt ist. Hierbei ist zwischen Reservoir und Haftkleberschicht eine die Wirkstofffreisetzung steuernde Membran angebracht.

Die Dicke des transdermalen Pflasters richtet sich nach den therapeutischen Erfordernissen und kann entsprechend angepaßt werden. Sie liegt üblicherweise im Bereich von 0,03-0,4 mm.

Eine bevorzugte Einsatzform stellt weiterhin ein monolithisches Matrix-Transdermales therapeutisches System dar, das aus einer im wesentlichen wirkstoffundurchlässigen Rückschicht, der eigentlich aktiven Matrix-Schicht (welche die erfindungsgemäßen Wirk- und Hilfsstoffe enthält) sowie einer wiederablösbaren Schutzschicht besteht.

Wie in den Beispielen gezeigt, weisen solche Systeme bei gegenüber dem Stand der Technik vereinfachtem Aufbau und geringerem Aufwand verbesserte, auch gleichmäßigere, Permeationsdaten beider Wirkstoffe auf.

Es hat sich überraschend gezeigt, daß eine solche, aus vorwiegend lipophilen sowie vergleichsweise schwach diffusiblen Polymeren und Harzen aufgebaute Rezeptur zu Human-Blutspiegeln führt, die sich mit Systemen nach dem Stand der Technik bei vergleichbar geringem Aufwand nicht erzeugen lassen.

Kautschukkleber galten bisher als weniger geeignet für die Abgabe von Estradiol an die Haut. So wird dem in EP 0 186 019 beschriebenen Gedanken, Kautschukkleber (hier unter Zusatz von wasserquellbaren Stoffen) zu verwenden, in EP 0 421 454 (S. 2, Z. 54 f.) widersprochen: eine ausreichende Freisetzung von Estradiol sei in diesen gering diffusiblen und nur schwach lösenden Polymeren nicht gegeben.

Beide nach der Erfindung wesentlichen Einsatzstoffe, Styrol-Isopren-Blockcopolymer und hydrierte Harzsäuren bzw. deren Derivate, sind seit langer Zeit als klassische Grundmaterialien von Heftpflastern erfolgreich im Einsatz und weisen gut Verträglichkeit auf. Unter der Begriff "Hydrierte Harzsäuren" werden vom Naturprodukt "Kolophonium" abgeleitete Verbindungen verstanden. Kolophonium findet als Gemisch nativer Harzsäuren vor allem in chemisch modifizierter Form breite Verwendung in Bedarfsgegenständen, Kosmetika, Lebensmittelverpackungen, Kaugummi etc.. Dabei handelt es sich um den nach Abdestillieren von Terpentinöl verbleibenden harzigen Rückstand des Rohproduktes Terpentinbalsam, der von verschiedenen Kiefernarten aus überwiegend subtropisch-mediterranen Klimazonenstammt.

Das Rohprodukt ist eine spröde, harzartige, bei ca. 73-80°C erweichende Masse mit einer Dichte um 1,07 g/ml. Die Modifizierung des Kolophoniums für den Zweck der Verwendung in transdermalen Systemen dient der Stabilisierung gegen Sauerstoffeinflüsse durch Hydrierung sowie der Verbesserung der Alkalistabilität durch Veresterung. Durch Hydrierung und gegebenenfalls Derivatisierung wird das Material für den vorgesehenen Zweck geeigneter gemacht. Wichtige, für den erfindungsgemäßen Zweck verwendbare Ester sind z.B. Glycerinester, Pentaerythritester, Methylester sowie auch andere hautverträgliche Derivate des hydrierten Kolophoniums.

Synthetische Kautschukpolymere spielen eine bedeutende Rolle bei der Herstellung von transdermalen therapeutischen Systemen wie auch von Wundpflastern. Ihr Vorteil liegt in einer erheblichen Verbesserung der mechanischen Eigenschaften eines transdermalen therapeutischen Systems. In dieser Hinsicht haben sich die Styrol-Isopren-Styrol-Blockcopolymere besonders bewährt. Durch das Unterteilen der Polymerkette in einen Mittelblock aus noch beweglichen langekettigen Polyisopreneinheiten und die beiden Polystyrolenden als "Ankerpunkte" entsteht in der Matrix ein dreidimensionales Netzwerk, das für weitgehend konstante Geometrie auch während der Lagerung sorgt. Dabei ist es im einzelnen nicht entscheidend, welches Molekulargewicht oder welches Verhältnis zwischen dem Anteil der Styroldomänen und der Polyisoprendomänen nun tatsächlich vorliegt. Wichtig ist vielmehr die Einstellung der korrekten Klebrigkeit und Kohäsion. Beispeilsweise bewirkt ein erhöhter Harzanteil verbesserte Klebrigkeit auf der Haut, aber auch weichere Konsistenz der Matrix. In der Regel wird man mit einem Anteil von rund einem Drittel des Blockcopolymers rechnen, der nach Zusatz der Wirkstoffe verbleibende Rest sind biokompatible Harzderivate.

Auch wenn ein einschichtiger Aufbau des transdermalen therapeutischen Systems wegen der Einfachheit der Funktion Vorteile aufweist, ist es ohne weiteres möglich, ein solches Matrixsystem erfindungsgemäß zum Beispiel mit einer dünnen, zusätzlichen Klebschicht zur Haut hin zu versehen. Auch läßt sich zur besseren Verankerung auf der Rückschicht eine dünne Haftschicht auflaminieren. Solche Zusatzschichten können aus einem Kautschuk-Harz-Gemisch oder aber auch z.B. acrylsäureesterhaltigen Copolymeren bestehen. Sie können auch dann verwendet werden, wenn sie vor dem Laminieren keine Beladung mit Wirkstoffen erhalten haben, da beim kurzzeitigen Zwischenlagern des Gesamtlaminates ein Diffusionsausgleich stattfindet.

Im folgenden wird die Erfindung anhand von Beispielen weiter erläutert.

### Beispiel 1:

- 73,1 g: Triethylenglycolester von hydriertem Kolophonium (Staybelite Ester 3E/Fa. Hercules) und
- 9,8 g: Glycerinester von hydriertem Kolophonium (Staybelite Ester 10E/Fa Hercules)
werden bei 100°C 5 Minuten durch Kneten gemischt. Anschließend werden 2,5 g Estradiol zugegeben. Es wird 30 Minuten geknetet. Nach Aufheizen auf 140°C werden 14,6 g Ethylcellulose N50NF (Fa. Hercules) portionsweise zugegeben und anschließend noch 2,5 St. geknetet.

Die so erhaltene wirkstoffhaltige Klebmasse wird mit einer Hotmelt-Beschichtungsanlage (Düsenauftragssystem) so auf eine wiederablösbare Schutzschicht (Hostaphan RN 100 einseitig mit Silikon beschichtet - Fa. Kalle ) beschichtet, daß ein wirkstoffhaltiges Reservoir mit einem Flächengewicht von 80 g/m² resultiert. Auf dieses Reservoir wird die undurchlässige Rückschicht (Polyesterfolie, 15 µm dick) aufkaschiert. Anschließend werden 16 cm²-große Wirkstoffpflaster gestanzt.

### Beispiel 2:

Die Herstellung erfolgt wie unter Beispiel 1 beschrieben, wobei der Weichmacher mit den beiden Staybelite Estern 3E und 10E gemeinsam geknetet wird.

### Beispiele 3-9:

Die Herstellung erfolgt wie unter Beispiel 1 beschrieben, jedoch mit den in Tabelle 1 (Herstellformel) angegebenen Rohstoffen und Mengen.

### Analytik:

Die Wirkstofffreisetzung der 16 cm²-großen transdermalen Pflaster wird nach der in der USP XXII beschriebenen Rotating bottle-Methode in 0,9%iger Kochsalzlösung bei 37°C bestimmt.

Zur Messung der Mäusehauptpenetration wird die Haut von haarlosen Mäusen in die Franz-Zelle eingespannt. Auf die Haut wird ein oestradiolhaltiges Pflaster mit einer Fläche von
2,54 cm² aufgeklebt und die Wirkstofffreisetzung bei 37°C (Akzeptormedium: 0,9%ige Kochsalzlösung) gemessen (Literatur: Umesh V. Banakar Pharmaceutical dissolution testing (1. Auflage - 1991)).

Die Prüfung auf Rekristallisationserscheinung wird visuell im Gegenlicht durchgeführt.

Die Ergebnisse sind in Tabelle 2 dargestellt.

**Tabelle 1**

| Herstellformel (Angaben in g) | | | | | | |
|---|---|---|---|---|---|---|
| Bsp. | Ethylcellulose N50NF | Staybelite Ester | | Weichmacher Miglyol 812 | Estradiol | Antioxidantien |
| | | 3E | 10E | | | |
| 1 | 14,6 | 73,1 | 9,8 | --- | 2,5 | |
| 2 | 14,3 | 71,6 | 9,6 | 2,0 | 2,5 | |
| 3 | 10,1 | 75,4 | 10,0 | 2,0 | 2,5 | |
| 4 | 7,7 | 77,5 | 10,3 | 2,0 | 2,5 | |
| 5 | 14,3 | 71,6 | 9,5 | 2,0 | 2,5 | 0,1 BHT |
| 6 | 14,3 | 71,6 | 9,5 | 2,0 | 2,5 | 0,1 BHA |
| 7 | 14,3 | 71,6 | 9,5 | 2,0 | 2,5 | 0,1 BHT:BHA =1 : 1 |
| 8 | 14,3 | 71,6 | 9,6 | 2,0 Isopropylpalmitat | 2,5 | |
| 9 | 14,3 | 71,6 | 9,5 ⊗ | 2,0 | 2,5 | |
| BHT = Butylhydroxitoluol BHA = Butylhydroxianisol ⊗ = Foral 105 (Pentaerythritester von hydriertem Kolophonium) | | | | | | |

**Tabelle 2**

| Analysenergebnisse | | | | |
|---|---|---|---|---|
| Bsp. | Estradiolgehalt µg/16cm² | in vitro-Freisetzung µg/16cm² · 24 Std. | Mäusehautpenetration µg/16cm² · 24 Std. | Rekristallisation |
| 1 | 3200 | 614 | 225 | keine |
| 2 | 3200 | 1240 | 300 | " |
| 3 | 3200 | 722 | 235 | " |
| 4 | 3200 | 713 | 268 | " |
| 5 | 3200 | 624 | 228 | " |
| 6 | 3200 | 624 | 249 | " |
| 7 | 3200 | 620 | 205 | " |
| 8 | 3200 | 686 | 232 | " |
| 9 | | | | |
| nach DE 3933460 | 3200 | 2400 | 125 | erheblich |
| Wie die Tabelle zeigt, erhält man eine deutlich bessere Penetration durch die Mäusehaut wie das Vergleichsbeispiel unter DE 3933460 unter Beweis stellt. Parallel dazu kann festgestellt werden, daß die Rekristallisation der erfindungsgemäßen Beispiele völlig unterbleibt. | | | | |

### Beispiel 10:

1,0 g 17-β-Estradiol
1,3 g Levonorgestrel
60,0 g Cariflex^{R} TR 1107 (Styrol-Isopren-Styrol-Blockcopolymer) 138,0 g Foral^{R} 85 (thermoplastisches Esterharz aus Kolophoniumderivaten)
200,0 g Benzin (Siedebereich 80-100°C)
werden bei Raumtemperatur in einem zylindrischen Glasgefäß bis zum Erreichen einer gleichmäßigen Suspension gerührt und anschließend auf einer kontinuierlich arbeitenden Beschichtungsanlage auf eine 100 Mikrometer dicke, siliconisierte Polyesterfolie so beschichtet, daß eine Schichtdicke von 110 g/m² (bezogen auf den lösemittelfreien Anteil) resultiert. Der Ausstrich wird jeweils 3 Minuten lang bei 40 Grad C, 60 Grad C, 75 Grad C und bei 125 Grad C getrocknet. Sofort wird eine 12 Mikrometer dicke Polyesterfolie luftblasenfrei unter Walzendruck auf die getrocknete Schicht aufgelegt (zukaschiert). Durch Stanzung mit Henkellocheisen werden transdermale Systeme von 20 cm² erhalten.

### Beispiel 11: Herstellung eines erfindungsgemäßen Systems

1,5 g 17-β-Estradiol
1,5 g Levonorgestrel
70,0 g Styrol-Isopren-Styrol-Blockcopolymer
150,0 g thermoplastisches Esterharz aus Kolophoniumderivaten
werden in einem beheizbaren Kneter bei 150°C unter Stickstoff innerhalb von 24h verschmolzen und knetend vereinigt. Auf einer kontinuierlich arbeitenden Beschichtungsanlage wird in einer Schichtdicke von 100 Mikrometer eine 19 µm dicke Polyesterfolie mit der Schmelze beschichtet. Dies kann bei 140°C in einem Schmelzauftragswerk oder auch bei ca. 80-100°C mittels eines Extruders geschehen. Anschließend wird eine 150 Mikrometer dicke, siliconisierte Polyesterfolie, vorbeschichtet mit 20 g/m² eines Acrylestercopolymers (Durotak^{R} 280-2516), luftblasenfrei unter Walzendruck auf die getrocknete Schicht aufgelegt (zukaschiert). Durch Stanzung mit Henkellocheisen werden transdermale Systeme von 20 cm² erhalten.

## Patentansprüche

1. Transdermales therapeutisches System, enthaltend als Wirkstoff zur kontrollierten Abgabe von Estradiol oder dessen pharmazeutisch unbedenklichen Derivaten allein oder in Kombination mit Gestagenen, insbesondere Levonorgestrel, bestehend aus einer Rückschicht und einem unter Verwendung von Haftklebern hergestellten mit der Rückschicht verbundenen, den Wirkstoff enthaltenden, aus einem oder mehreren Schichten bestehenden Reservoir, dadurch gekennzeichnet, daß der Haftkleber Ester des Kolophoniums oder Ester des hydrierten Kolophoniums mit einem Anteil von 55-92 Gew.-% sowie zusätzliche organische Polymere enthält, welche
a) falls der alleinige Wirkstoff Estradiol oder einer seiner pharmazeutisch unbedenklichen Derivate ist, als Celluloseether wie Ethylcellulose vorliegen,
oder
b) falls der Wirkstoff aus der Kombination Estradiol oder eines seiner pharmazeutisch unbedenklichen Derivate mit einem Gestagen, insbesondere Levonorgestrel besteht, als synthetische Kautschukpolymere vorliegen.

2. Transdermales therapeutisches System noch Anspruch 1b, dadurch gekennzeichnet, daß die synthetischen Kautschukpolymere Polymere aus der Gruppe der Styrol-Butadien-Styrol, Styrol-Isopren-Styrol, Styrol-Ethylen-Butylen-Styrol-Blockpolymere umfassen.

3. Transdermales therapeutisches System nach Anspruch 1b oder 2, dadurch gekennzeichnet, daß der Anteil der synthetischen Kautschukpolymere in der aktiven Schicht 10-45 Gew.-%, vorzugsweise 15-33 Gew.-% beträgt.

4. Transdermales therapeutisches System nach einem oder mehreren der vorstehenden Ansprüche 1b bis 3, dadurch gekennzeichnet, daß das Reservoir Estradiol oder seine pharmazeutisch unbedenklichen Derivate in Kombination mit Gestagenen in einer Konzentration von insgesamt 2-15 Gew.-% und zwar in einem molaren Verhältnis 1:1 bis 1:10 enthält.

5. Transdermales therapeutisches System nach einem oder mehreren der Ansprüche 1b bis 4, dadurch gekennzeichnet, daß die Konzentration an Levonorgestrel in der aktiven Schicht zwischen 0,1 und 1,6 Gewichtsanteilen liegt.

6. Transdermales therapeutisches System nach einem der Ansprüche 1-5, dadurch gekennzeichnet, daß der Haftkleber Ester des Kolophoniums in einem Anteil zwischen 60 und 90 Gew.-% enthält.

7. Transdermales therapeutisches System nach Anspruch 6, dadurch gekennzeichnet, daß der Haftkleber Ester des Kolophoniums in einem Anteil zwischen 70 und 88 Gew.-% enthält.

8. Transdermales therapeutisches System nach Anspruch 1b, dadurch gekennzeichnet, daß Ester des gegebenenfalls hydrierten Kolophoniums aus der Gruppe bestehend aus Methylester, Glycerinester, Pentaerythritester, maleinsäuremodifiziertem Pentaerithritester und maleinsäuremodifiziertem Glycerinester ausgewählt sind.

9. Transdermales therapeutisches System nach einem oder mehreren der vorstehenden Ansprüche, dadurch gekennzeichnet, daß Triethylenglycolester des gegebenenfalls hydrierten Kotophoniums in Reservoir enthalten ist.

10. Transdermales therapeutisches System nach einem oder mehreren der vorstehenden Ansprüche, dadurch gekennzeichnet, daß die Konzentration an Estradiol in der aktiven Schicht zwischen 0,2 und 2 Gewichtsanteilen, bevorzugt zwischen 0,7 und 1,4 Gewichtsanteilen liegt.

11. Transdermales therapeutisches System nach einem oder mehreren der vorstehenden Ansprüche, dadurch gekennzeichnet, daß die Schichtdicke der aktiven Schicht zwischen 30 und 300 µm, bevorzugt zwischen 70 und 120 µm liegt.

12. Transdermales therapeutisches System nach einem oder mehreren der vorstehenden Ansprüche, dadurch gekennzeichnet, daß es den oder die Wirkstoffe Levonorgestrel und Estradiol teilweise in Suspension enthält.

14. Transdermales therapeutisches System nach Anspruch 1a, dadurch gekennzeichnet, daß der Haftkleber als weitere Bestandteile Polymere aus der Gruppe der Ethylen-Vinylacetat-Copolymere, Polyvinylpyrrolidon und Cellulosederivate und Polymere auf der Basis von Acrylsäure- und Methacrylsäurederivaten enthält.

15. Transdermales therapeutisches System nach einem oder mehreren der vorstehenden Ansprüche, dadurch gekennzeichnet, daß das Reservoir mindestens einen Bestandteil aus der Gruppe, bestehend aus Alterungsschutzmitteln, Weichmachern, Antioxidantien und Absorptionsverbesserern, enthält, wobei der Weichmacher in einer Konzentration von 0-5 Gew.-% und das Alterungsschutzmittel in einer Konzentration von 0,1 Gew.-% enthalten ist.

16. Transdermales therapeutisches System nach einem oder mehreren der vorstehenden Ansprüche, dadurch gekennzeichnet, daß der Haftkleber ein Lösemittelhaftkleber ist.

17. Transdermales therapeutisches System nach einem oder mehreren der vorstehenden Ansprüche, dadurch gekennzeichnet, daß der Haftkleber ein Schmelzhaftkleber ist.

18. Transdermales therapeutisches System nach einem oder mehreren der vorstehenden Ansprüche, dadurch gekennzeichnet, daß das Reservoir mit einer zusätzlichen haftklebenden Schicht bzw. mit einem haftklebenden Rand versehen ist.

19. Transdermales therapeutisches System nach einem oder mehreren der vorstehenden Ansprüche, dadurch gekennzeichnet, daß zwischen Reservoir und Haftklebeschicht eine die Wirkstoff-Freisetzung steuernde Membran angebracht ist.

20. Verfahren zur Herstellung eines transdermalen therapeutischen Systems nach einem oder mehreren der vorstehenden Ansprüche, dadurch gekennzeichnet, daß es die folgenden Schritte umfaßt:
Kneten der Mischung aus Estern des Kolophoniums bei erhöhter Temperatur bis zur Homogenisierung, Einarbeitung von Wirkstoffen und mindestens einem Polymer bei Lösetemperatur, nach Homogenisierung Beschichten einer wiederablösbaren Schutzschicht mit der wirkstoffhaltigen Klebmasse und Aufkaschieren der Rückschicht.

## Claims

1. Transdermal therapeutic system containing as active substance for controlled release estradiol or its pharmaceutically acceptable derivatives, alone or in combination with gestagens, especially with levonorgestrel, consisting of a backing layer and of a reservoir, which reservoir has been produced using pressure-sensitive adhesives, is connected with the backing layer, contains the active substance, and consists of one or more layers, characterized in that the pressure-sensitive adhesive contains esters of colophony or esters of hydrogenated colophony at a proportion of 55 - 92%-wt., as well as additional organic polymers which
a) if the sole active substance is estradiol or one of its pharmaceutically acceptable derivatives, are present as cellulose ethers, such as ethyl cellulose,
or
b) if the active substance consists of a combination of estradiol or one of its pharmaceutically acceptable derivatives with a gestagen, especially with levonorgestrel, are present as synthetic rubber polymers.

2. Transdermal therapeutic system according to claim 1b, characterized in that the synthetic rubber polymers comprise polymers from the group of styrene-butadiene-styrene, styrene-isoprene-styrene, styrene-ethylene-butylene-styrene block polymers.

3. Transdermal therapeutic system according to claim 1b or 2, characterized in that the proportion of synthetic rubber polymers in the active layer is 10-45%-wt., preferably 15-33%-wt.

4. Transdermal therapeutic system according to one or more of the preceding claims 1b to 3, characterized in that the reservoir comprises estradiol or its pharmaceutically acceptable derivatives in combination with gestagens at a concentration totalling 2 - 15%-wt., that is at a molar ratio of 1:1 to 1:10.

5. Transdermal therapeutic system according to one or more of claims 1b to 4, characterized in that the concentration of levonorgestrel in the active layer is between 0.1 and 1.6 parts by weight.

6. Transdermal therapeutic system according to one of claims 1 - 5, characterized in that the pressure-sensitive adhesive contains esters of colophony at a proportion between 60 and 90%-wt.

7. Transdermal therapeutic system according to claim 6, characterized in that the pressure-sensitive adhesive contains esters of colophony at a proportion between 70 and 88%-wt.

8. Transdermal therapeutic system according to claim 1b, characterized in that esters of colophony, which optionally is hydrogenated, are selected from the group consisting of methyl ester, glycerol ester, pentaerythritol ester, pentaerythritol ester modified with maleic acid, and glycerol ester modified with maleic acid.

9. Transdermal therapeutic system according to one or more of the preceding claims, characterized in that triethylene glycol ester of the, optionally hydrogenated, colophony is contained in the reservoir.

10. Tranadermal therapeutic system according to one or more of the preceding claims, characterized in that the concentration of estradiol in the active layer amounts to between 0.2 and 2 parts by weight, preferably between 0.7 and 1.4 parts by weight.

11. Transdermal therapeutic system according to one or more of the preceding claims, characterized in that the layer thickness of the active layer amounts to between 30 and 300 µm, preferably between 70 and 120 µm.

12. Transdermal therapeutic system according to one or more of the preceding claims, characterized in that it contains the active substance or active substances, levonorgestrel and estradiol, partially in suspension.

13. Transdermal therapeutic system according to claim 1a, characterized in that the pressure-sensitive adhesive contains, as further components, polymers of the group of ethylene-vinyl acetate copolymers, polyvinylpyrrolidone and cellulose derivatives, as well as polymers based on acrylic acid derivatives and methacrylic acid derivatives.

14. Transdermal therapeutic system according to one or more of the preceding claims, characterized in that the reservoir comprises at least one component from the group consisting of anti-ageing agents, plasticizers, anti-oxidants, and absorption improvers, with the plasticizer being contained at a concentration of 0 - 5%-wt. and the anti-ageing agent being contained at a concentration of 0.1%-wt.

15. Transdermal therapeutic system according to one or more of the preceding claims, characterized in that the pressure-sensitive adhesive is a solvent-based pressure-sensitive adhesive.

16. Transdermal therapeutic system according to one or more of the preceding claims, characterized in that the pressure-sensitive adhesive is a hot-melt pressure-sensitive adhesive.

17. Transdermal therapeutic system according to one or more of the preceding claims, characterized in that the reservoir is provided with an additional pressure-sensitive layer or with a pressure-sensitive adhesive edge.

18. Transdermal therapeutic system according to one or more of the preceding claims, characterized in that a membrane which controls the active substance release is located between the reservoir and the pressure-sensitive adhesive layer

19. Process for the production of a transdermal therapeutic system according to one or more of the preceding claims, characterized in that it comprises the following steps: kneading the mixture of esters of colophony at an elevated temperature until homogenization, incorporating active substances and at least one polymer at the solution temperature, coating a removable protective layer with the active substance-containing adhesive mass after homogenization, and laminating the backing layer.

## Revendications

1. Système thérapeutique transdermique pour le dégagement contrôlé d'oestradiol ou de ses dérivés pharmaceutiquement acceptables, seul(s) ou en combinaison avec des progestatifs, en particulier le lévonorgestrel, qu'il contient à titre de principe actif, constitué par une couche dorsale et par un réservoir préparé en utilisant des agents auto-adhésifs, relié à la couche dorsale, contenant le principe actif et constitué par une ou plusieurs couches, caractérisé en ce que l'agent auto-adhésif contient des esters du colophane ou des esters du colophane hydrogéné en une fraction représentant de 55 à 92% en poids, ainsi que des polymères organiques supplémentaires qui
a) au cas où le principe actif à titre individuel est constitué d'oestradiol ou d'un de ses dérivés pharmaceutiquement acceptables, sont présents sous la forme d'éthers cellulosiques tels que l'éthylcellulose,
ou
b) au cas où le principe actif est constitué par la combinaison d'oestradiol ou d'un de ses dérivés pharmaceutiquement acceptables avec un progestatif, en particulier le lévonorgestrel, sont présents sous forme de polymères de caoutchoucs synthétiques.

2. Système thérapeutique transdermique selon la revendication 1b, caractérisé en ce que les polymères de caoutchoucs synthétiques sont des polymères choisis parmi le groupe comprenant des copolymères séquencés de styrène-butadiène-styrène, de styrène-isoprène-styrène, de styrène-éthylène-butylène-styrène.

3. Système thérapeutique transdermique selon la revendication 1b ou 2, caractérisé en ce que la fraction des polymères de caoutchoucs synthétiques dans la couche active représente de 10 à 45% en poids, de préférence de 15 à 33% en poids.

4. Système thérapeutique transdermique selon une ou plusieurs des revendications précédentes 1b à 3, caractérisé en ce que le réservoir contient de l'oestradiol ou ses dérivés pharmaceutiquement acceptables en combinaison avec des progestatifs on une concontration au total de 2 à 15% en poids et en fait dans un rapport molaire de 1:1 à 1:10.

5. Système thérapeutique transdermique selon une ou plusieurs des revendications 1b à 4, caractérisé en ce que la concentration du lévonorgestrel dans la couche active se situe entre 0,1 et 1,6 partie en poids.

6. Système thérapeutique transdermique selon l'une quelconque des revendications 1 à 5, caractérisé on ce que l'agent auto-adhésif contient des esters du colophane on une fraction entre 60 et 90% en poids.

7. Système thérapeutique transdermique selon la revendication 6, caractérisé en ce que l'agent auto-adhésif contient des esters du colophane en une fraction entre 70 et 88% en poids.

8. Système thérapeutique transdermique selon la revendication 1b, caractérisé on ce que des esters du colophane le cas échéant hydrogéné sont choisis parmi le groupe constitué par l'ester méthylique, l'ester de glycérol, l'ester de pentaérythritol, l'ester de pentaérythritol modifié avec de l'acide maléique et l'ester de glycérol modifié avec de l'acide maléique.

9. Système thérapeutique transdermique selon une ou plusieurs des revendications précédentes, caractérisé en ce que de l'ester de triéthylèneglycol du colophane le cas échéant hydrogéné est contenu dans le réservoir.

10. Système thérapeutique transdermique selon une ou plusieurs des revendications précédentes, caractérisé en ce que la concentration d'oestradiol dans la couche active se situe entre 0,2 et 2 parties en poids, de préférence entre 0,7 et 1,4 partie on poids.

11. Système thérapeutique transdermique selon une ou plusieurs des revendications précédentes, caractérisé en ce que l'épaisseur de la couche active se situe entre 30 et 300 µm, de préférence entre 70 et 120 µm.

12. Système thérapeutique transdermique selon une ou plusieurs des revendications précédentes, caractérisé en ce qu'il contient le ou les principes actifs de lévonorgestrel et d'oestradiol en partie en suspension.

13. Système thérapeutique transdermique selon la revendication 1a, caractérisé en ce que l'agent auto-adhésif contient, à titre de constituants supplémentaires, des polymères choisis parmi le groupe comprenant des copolymères d'éthylène-acétate de vinyle, la polyvinylpyrrolidone et des dérivés de cellulose, ainsi que des polymères à base de dérivés d'acide acrylique et d'acide méthacrylique.

14. Système thérapeutique transdermique selon une ou plusieurs des revendications précédentes, caractérisé en ce que le réservoir contient au moins un constituant choisi parmi le groupe constitué par des agents de protection contre le vieillissement, des plastifiants, des antioxydants et des agents améliorant l'absorption, le plastifiant étant contenu en une concentration de 0 à 5% en poids et l'agent de protection contre le vieillissement étant contenu en une concentration de 0,1% en poids.

15. Système thérapeutique transdermique selon une ou plusieurs des revendications précédentes, caractérisé en ce que l'agent auto-adhésif est un agent auto-adhésif contenant un solvant.

16. Système thérapeutique transdermique selon une ou plusieurs des revendications précédentes, caractérisé en ce que l'agent auto-adhésif est un agent auto-adhésif à fusion.

17. Système thérapeutique transdermique selon une ou plusieurs des revendications précédentes, caractérisé en ce que le réservoir est muni d'une couche auto-adhésive supplémentaire, respectivement d'un bord auto-adhésif.

18. Système thérapeutique transdermique selon une ou plusieurs des revendications précédentes, caractérisé en ce qu'on applique, entre le réservoir et la couche auto-adhésive, une membrane réglant la libération du principe actif

19. Procédé pour la fabrication d'un système thérapeutique transdermique selon une ou plusieurs des revendications précédentes, caractérisé en ce qu'il comprend les étapes ci-après:
malaxage du mélange d'esters du colophane à une température élevée jusqu'à l'homogénéisation; incorporation de principes actifs et d'au moins un polymère à la température de dissolution; après homogénéisation, enduction d'une couche de protection pelliculable avec la matière adhésive contenant le principe actif; et contrecollage de la couche dorsale.
